(19) Europäisches Patentamt
European Patent Office
Office européen des brevets

(11) Publication number: **0 003 866**
**B1**

(12) **EUROPEAN PATENT SPECIFICATION**

(45) Date of publication of patent specification: **04.11.81**

(51) Int. Cl.³: **C 07 D 209/52**

(21) Application number: **79200095.2**

(22) Date of filing: **27.02.79**

(54) **3-Azabicyclo(3.1.0)hexane derivatives and a process for their preparation.**

(30) Priority: **01.03.78 GB 806678**

(43) Date of publication of application:
**05.09.79 Bulletin 79/18**

(45) Publication of the grant of the European patent:
**04.11.81 Bulletin 81/44**

(84) Designated Contracting States:
**BE CH DE FR GB IT NL SE**

(56) References cited:
**FR - A - 2 324 236**

**CHEMICAL ABSTRACTS, vol. 77, no. 1, July 3, 1972, Columbus Ohio, USA I. ROWLAND et al.: "Inhibition of bacterial growth by cis- and trans-3,4-methano-1-proline", page 110, abstract 14817y.**

(73) Proprietor: **SHELL INTERNATIONALE RESEARCH MAATSCHAPPIJ B.V.**
**Carel van Bylandtlaan 30**
**NL-2596 HR DEN HAAG (NL)**

(72) Inventor: **Mason, Ronald Frank**
**"Kingswood" Westwell**
**nr Ashford Kent (GB)**
Inventor: **Devlin, Barry Roy John**
**59 Ruins Barn Road**
**Sittingbourne Kent (GB)**

(74) Representative: **Keuzenkamp, Abraham et al,**
**c/o Shell Internationale Research Maatschappij B.V. P.O. Box 302**
**NL-2501 CH 's-Gravenhage (NL)**

**EP 0 003 866 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

# 3-Azabicyclo (3.1.0) hexane derivatives and a process for their preparation

The present invention relates to 3-azabicyclo [3.1.0] hexane derivatives and to a process for their preparation. These compounds are useful starting materials for the preparation of 2-cyano-3-azabicyclo [3.1.0] hexane derivatives.

The present invention provides a compound of the general formula

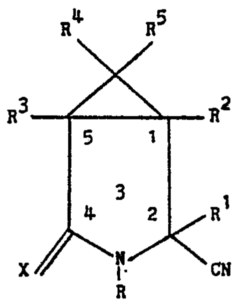

(I)

wherein R represents a hydrogen atom or an alkyl or cycloalkyl group which may be unsubstituted or substituted by one or more of the same or different substituents selected from halogen atoms, alkoxy groups and aryl groups; $R^1$ represents a hydrogen atom or an alkyl or cycloalkyl group which may be unsubstituted or substituted by one or more of the same or different substituents selected from halogen atoms and alkoxy groups; each of $R^2$, $R^3$, $R^4$ and $R^5$ independently represents a hydrogen or halogen atom or an alkyl, cycloalkyl, alkenyl, aryl, alkaryl or aralkyl group which may be unsubstituted or substituted by one or more of the same or different substituents selected from halogen atoms and alkoxy groups, or each of $R^2$ and $R^3$ independently has one of the above meanings and $R^4$ and $R^5$ together represent an alkylene group; and X represents an oxygen or sulphur atom.

Unless otherwise stated, any halogen atom present in a compound according to the invention is preferably a chlorine or fluorine atom. Any alkyl, alkenyl, or alkoxy group preferably has up to 6, especially up to 3, carbon atoms. Any aryl moiety is preferably a phenyl moiety, and any alkaryl or aralkyl group preferably has 7 or 8 carbon atoms.

R may for example represent a hydrogen atom or an alkyl group which may be unsubstituted or substituted by one or more of the same or different substituents selected from halogen atoms, alkoxy groups and aryl groups. Preferably however, R represent a hydrogen atom, an alkyl group having up to 6 carbon atoms which may be unsubstituted or substituted by up to 3 halogen atoms, a cycloalkyl group, or a benzyl group which may be unsubstituted or substituted in the ring by up to 3 halogen atoms. More preferably, R represents a hydrogen atom or an alkyl group having up to 4 carbon atoms. Most preferably, R represents a methyl group or, especially, a hydrogen atom.

Preferably $R^1$ represents a hydrogen atom or an alkyl group having up to 6 carbon atoms which may be unsubstituted or substituted by up to 3 halogen atoms. More preferably $R^1$ represents a hydrogen atom or an alkyl group having up to 4 carbon atoms. Most preferably, $R^1$ represents a methyl group or, especially, a hydrogen atom.

Preferably each of $R^2$, $R^3$, $R^4$ and $R^5$ independently represents a hydrogen or halogen atom, an alkyl or alkenyl group having up to 6 carbon atoms which may be unsubstituted or substituted by up to 3 halogen atoms, or an aryl, aralkyl or alkaryl group having up to 10 carbon atoms which may be unsubstituted or substituted by up to 3 halogen atoms, or each of $R^2$ and $R^3$ independently has one of these meanings and $R^4$ and $R^5$ together represent an alkylene group having up to 6 carbon atoms. More preferably, each of $R^2$, $R^3$, $R^4$ and $R^5$ independently represents a hydrogen, fluorine, chlorine or bromine atom or an alkyl or alkenyl group having up to 4 carbon atoms which may be unsubstituted or substituted by up to 3 fluorine, chlorine or bromine atoms, for example a methyl, isobutenyl, difluorovinyl or dichlorovinyl group, or each of $R^2$ and $R^3$ independently has one of these meanings and $R^4$ and $R^5$ together represent an alkylene group having 4 or 5 carbon atoms. Most preferably, each of $R^2$, $R^3$, $R^4$ and $R^5$ represents a hydrogen atom.

Preferably X represents an oxygen atom.

An especially preferred compound according to this invention is 2-cyano-3-azabicyclo [3.1.0] hexan-4-one.

The invention also provides a process for the preparation of a compound of the general formula I characterised in that a compound of the general formula

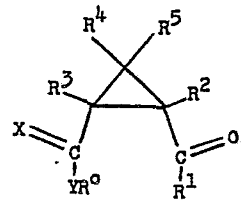

(II)

in which X, $R^1$, $R^2$, $R^3$, $R^4$ and $R^5$ have the meanings given for the general formula I, Y represents an oxygen or sulphur atom, $R^o$ represents an optionally substituted alkyl group, an alkali metal ion, an ammonium ion optionally substituted by one or more alkyl groups, or one equivalent of an alkaline earth metal ion, and the groups $-COR^1$ and $-CXYR^o$ are in cis relationship to each other, is reacted with a cyanide in the presence of a compound of the general formula

$$RNH_2 \quad (III)$$

in which R has the meaning given for the general formula I.

The cyanide used in the process according to the invention may be hydrogen cyanide, a cyanide salt, or any compound which is capable of generating hydrogen cyanide in situ. Suitable cyanides include alkali metal cyanides, especially sodium and potassium cyanide, ammonium cyanide or an ammonium cyanide in which the ammonium ion is substituted by one or more alkyl groups, especially tri- or tetramethylammonium cyanide,and cyanohydrins of aldehydes and ketones, for example the cyanohydrins derived from acetone, methyl ethyl ketone and acetone.

The process according to the present invention is however preferably carried out using hydrogen cyanide as a reactant. Conveniently, liquid hydrogen cyanide may be added to a cooled solution of the cyclopropyl derivative of formula II. When using a cyanide salt, such as sodium or potassium cyanide, the reaction is conveniently carried out in the additional presence of an ammonium halide, such as ammonium chloride or bromide.

Preferably the cyanide is used in a slight excess, e.g. an excess of 3 to 10% by weight, especially 4 to 7% by weight, based on the weight of cyclopropyl derivative of general formula II.

In the general formula II, $R^o$ is preferably an alkyl group having up to 6 carbon atoms, an alkali metal ion, or an ammonium ion optionally substituted by one or more alkyl groups having from 1 to 4 carbon atoms. More preferably, $R^o$ represents an alkali metal ion, an ammonium ion, or an alkyl group having up to 4 carbon atoms, for example a methyl or ethyl group. Preferably Y represents an oxygen atom.

An especially preferred compound of the general formula II is cis 1-ethoxycarbonyl-2-formylcyclopropane.

The compound of formula III is ether ammonia or a primary amine. Suitable primary amines include for example methylamine, ethylamine, isopropylamine, cyclohexylamine and benzylamine. Preference is however given to the use of ammonia, to produce a compound of the general formula I in which R represents a hydrogen atom.

The reactants in the process according to the invention may be added to each other in any sequence. For example, the compound of formula $RNH_2$ may be present in a solution containing the cyclopropyl derivative of formula II prior to the addition of the cyanide, or it can be added to a reaction mixture already containing a cyanide. It may be advantageous to add the ammonia or amine slowly and continuously to the reaction mixture during the course of the reaction. Good results may be obtained by saturating a cooled solution containing a cyclopropyl derivative of formula II and liquid hydrogen cyanide, with ammonia.

The process according to the invention is preferably carried out at a temperature of from 0 to 100°C. Suitably, the reaction is commenced at a temperature below 15°C, preferably below 5°C. The reaction is generally exothermic, and the reaction mixture may be allowed to reach ambient temperature. The reaction is preferably completed by heating the reaction mixture at a temperature of up to 80°C, preferably under reflux conditions.

A small amount of a base other than the compound of formula $RNH_2$, preferably a tertiary amine having up to 10 carbon atoms, e.g. triethyl amine or triethanolamine, or a secondary amine having up to 10 carbon atoms, e.g. piperidine or diethylamine, may also be present in the reaction mixture as it has a catalytic effect on the reaction. The amount of base added is preferably in the range 0.3 to 10% by weight, preferably 2 to 6% by weight, based on the weight of the cyclopropyl derivative of formula II.

The process according to the present invention can be carried out conveniently in a solvent. Suitable solvents include aliphatic alcohols, for example methanol, ethanol, isopropylalcohol, 2-chloroethanol and ethylene glycol, ethers, for example tetrahydrofuran, or aliphatic nitriles, for example acetonitrile. Ethanol is often the preferred solvent. Mixtures of two or more solvents may be used. In certain circumstances, it is possible to use an excess of one of the reagents, for example liquid ammonia or hydrogen cyanide, as a solvent or co-solvent.

The process according to the present invention is preferably carried out at atmospheric pressure.

If desired, superatmospheric pressures, e.g. up to 10 atmospheres, may be applied.

The compounds according to the invention exist in the form of geometric and optical isomers, the number of isomers depending on the meanings of the various substituents in the molecule. For example, the —CN group in the 2-position of the molecule may be cis or trans to the —$CR^4R^5$ group, and each of these isomers exists as a pair of optical isomers. The invention should be understood to include individual isomers and mixtures thereof.

The process according to the invention generally leads to mixture of isomers, which may if desired be separated into individual isomers or groups of isomers by conventional methods. For example, cis and trans isomers may be separated by fractional crystallisation or layer or column chromatography.

The compounds of the general formula II may be prepared by any suitable method, for example by the reaction of an olefinic compound and a sulphur ylid as described in U.S. Patent 3,397,223. Thus, 1-ethoxycarbonyl-2-formylcyclopropane may be prepared by adding acroleine to a solution of ethyl(dimethylsulphuranylidene) acetate in acetone.

The 3-azabicyclo [3.1.0]hexan-4-one derivatives according to formula I are valuable compounds for the synthesis of other compounds, as they possess various functional groups in the molecule. For example, they can be converted into the corresponding 2-cyano-3-azabicyclo [3.1.0] hexane derivatives, which have herbicidal properties and which are useful starting materials in the synthesis of certain 3,4-methanopyrrolidine derivatives which exhibit interesting pollen suppressant and plant growth regulating properties. Certain 3,4-methanopyrrolidine derivatives are described in German Offenlegungsschrift No. 2641295. The conversion of a compound according to the invention into the corresponding 2-cyano-3-azabicyclo [3.1.0] hexane derivative may be carried out as described in our co-pending application EP 4107 filed concurrently with the present application, by reacting the compound according to the present invention with a trialkyloxonium fluorobate, followed by reaction with a mild reducing agent, such as sodium hydride, lithium hydride, sodium borohydride or lithium borohydride.

The following Examples illustrate the invention.

## Example 1

Preparation of 2-cyano-3-azabicyclo [3.1.0] hexan-4-one

A 250 ml flask was charged with 21.3 g cis 1-ethoxycarbonyl-2-formylcyclopropane (0.15 mol), absolute ethanol (50 ml) and 4 drops of piperidine. The reaction contents were cooled to 0°C. Then 6 ml hydrogen cyanide (4.2 g; 0.155 mol) were added and the reaction mixture was saturated with anhydrous ammonia and allowed to attain room temperature. After the addition of further ammonia the temperature rose slowly and the mixture was kept at 70—75°C for 45 minutes. After removal of the volatile components in a film evaporator under reduced pressure, the remaining mixture was triturated with ethanol while cooling in an ice-bath, then filtered and recrystallised from 30 ml ethanol.

5.2 g of product, m.p. 135—136°C were obtained. This product was characterised by proton and $C^{13}$ nuclear magnetic resonance spectroscopy as the pure cis compound

| | | | | |
|---|---|---|---|---|
| Analysis: | Calculated for $C_6H_6N_2O$: | C 59.0; | H 5.0; | N 22.9% |
| | Found | C 59.1; | H 5.1; | N 23.0% |

From the mother liquor a further amount of 10.6 g 2-cyano-3-azabicyclo [3.1.0] hexan-4-one was obtained using chromatography over silica gel (methylene dichloride as eluent) as a cis/trans mixture. An analytically pure sample of the trans isomer (m.p. 89—90°C) was obtained using liquid-liquid chromatography. The compound was characterised by nuclear magnetic resonance spectroscopy. The total yield of products was 86%.

Similar results were obtained when the reaction was carried out using 0.675 mol of cis 1-ethoxycarbonyl-2-formylcyclopropane as starting material (66% of cis/trans product isolated).

## Example 2

Preparation of 2-cyano-3-azabicyclo [3.1.0] hexan-4-one starting with sodium cyanide and ammonium chloride

A 500 ml three-necked flask was charged with 22.2 g ammonium chloride (0.415 mol) and 20.0 g sodium cyanide (0.410 mol). The compounds were dissolved in 175 ml ammonia (s.g. 0.990) and 75 ml ethanol were added. The homogeneous solution thus obtained was then saturated with ammonia at 0—5°C. 26.7 g of cis 1-ethoxycarbonyl-2-formylcyclopropane (0.19 mol) were added over 5 minutes and the temperature raised to 25°C by warming the flask on a water bath. The reaction mixture was kept at this temperature for 5 hours and then worked up by removing the volatile components in a film evaporator at 60°C. 46 g of a solid mass were obtained. This product was extracted once with 150 ml of boiling ethanol and filtered. The inorganic residue (22.2 g) was discarded and filtrate evaporated to give 25 g of a semi-solid product which was taken up in 30 ml hot ethanol and allowed to crystallise.

4.8 g of pure cis 2-cyano-3-azabicyclo [3.1.0] hexan-4-one m.p. 135—137°C, were obtained. The product was identified as described in Example 1. From the mother liquor a further crop of 9.2 g of

cis/trans mixture was obtained (silica gel, ethanol/ methylene dichloride as eluents) so that a total yield of 73% was achieved.

**Claims**

1. A compound of the general formula

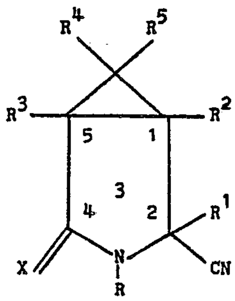

(I)

wherein R represents a hydrogen atom or an alkyl or cycloalkyl group which may be unsubstituted or substituted by one or more of the same or different substituents selected from halogen atoms, alkoxy groups and aryl groups; $R^1$ represents a hydrogen atom or an alkyl or cycloalkyl group which may be unsubstituted or substituted by one or more of the same or different substituents selected from halogen atoms and alkoxy groups; each of $R^2$, $R^3$, $R^4$ and $R^5$ independently represents a hydrogen or halogen atom or an alkyl, cycloalkyl, alkenyl, aryl, alkaryl or aralkyl group which may be unsubstituted or substituted by one or more of the same or different substituents selected from halogen atoms and alkoxy groups, or each of $R^2$ and $R^3$ independently has one of the above meanings and $R^4$ and $R^5$ together represent an alkylene group; and X represents an oxygen or sulphur atom.

2. A compound as claimed in claim 1, in which R represents a hydrogen atom, an alkyl group having up to 6 carbon atoms which may be unsubstituted or substituted by up to 3 halogen atoms, a cycloalkyl group, or a benzyl group which may be unsubstituted or substituted in the ring by up to 3 halogen atoms.

3. A compound as claimed in either claim 1 or claim 2, in which $R^1$ represents a hydrogen atom or an alkyl group having up to 6 carbon atoms which may be unsubstituted or substituted by up to 3 halogen atoms.

4. A compound as claimed in any one of claims 1 to 3, in which each of $R^2$, $R^3$, $R^4$, and $R^5$ independently represents a hydrogen, fluorine, chlorine or bromine atom or an alkyl or alkenyl group having up to 4 carbon atoms which may be unsubstituted or substituted by up to 3 fluorine, chlorine or bromine atoms, or each of $R^2$ and $R^3$ independently has one of these meanings and $R^4$ and $R^5$ together represent an alkylene group having 4 or 5 carbon atoms.

5. A compound as claimed in any one of claims 1 to 4, in which X represents an oxygen atom.

6. A compound as claimed in claim 1, which compound is 2-cyano-3-azabicyclo [3.1.0] hexan-4-one.

7. A process for the preparation of a compound as claimed in any one of claims 1 to 6, characterised in that a compound of the general formula

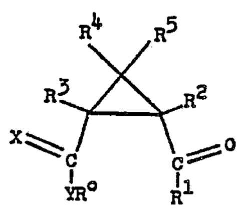

(II)

in which X, $R^1$, $R^2$, $R^3$, $R^4$ and $R^5$ have the meanings given for the general formula I, Y represents an oxygen or sulphur atom, $R^\circ$ represents an optionally substituted alkyl group, an alkali metal ion, an ammonium ion optionally substituted by one or more alkyl groups, or one equivalent of an alkaline earth metal ion, and the groups $-COR^1$ and $-CXYR^\circ$ are in cis relationship to each other, is reacted with a cyanide in the presence of a compound of the general formula

$$RNH_2 \qquad (III)$$

in which R has the meaning given for the general formula I of claim 1.

8. A process as claimed in claim 7, characterized in that the cyanide is hydrogen cyanide, an alkali metal cyanide, ammonium cyanide, an ammonium cyanide in which the ammonium ion is substituted by one or more alkyl groups, or a cyanohydrin of an aldehyde or ketone.

9. A process as claimed in either claim 7 or claim 8, characterized in that in the compound of the general formula II, R° represents an alkyl group having up to 6 carbon atoms, an alkali metal ion, or an ammonium ion optionally substituted by one or more alkyl groups having 1 to 4 carbon atoms, and Y represents an oxygen atom.

10. A process as claimed in any one of claims 7 to 9 characterized in that the reaction is carried out at a temperature in the range of from 0 to 100°C.

1. A process as claimed in any one of claims 7 to 10, characterized in that the reaction is conducted in the simultaneous presence of a base other than the compound of the general formula III.

## Revendications

1. Composé de la formule générale:

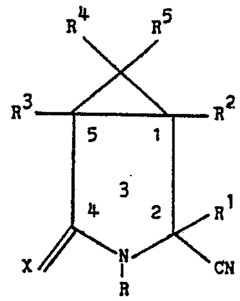

(I)

dans laquelle R représente un atome d'hydrogène ou un groupe alcoyle ou cycloalcoyle qui peut être non-substitué ou peut être substitué par un ou plusieurs substituants identiques ou différents choises parmi des atomes d'halogènes, des groupes alcoxy et des groupes aryle; $R^1$ représente un atome d'hydrogène ou un groupe alcoyle ou cycloalcoyle qui peut être non-substitué ou peut être substitué par un ou plusieurs substituants identiques ou différents choisis parmi des atomes d'halogènes et des groupes alcoxy; $R^2$, $R^3$, $R^4$ et $R^5$ représentent chacun indépendamment un atome d'hydrogène ou d'un halogène ou un groupe alcoyle, cycloalcoyle, alcényle, aryle, alcaryle ou aralcoyle qui peut être non-substitué ou peut être substitué par un ou plusieurs substituants identiques ou différents choisis parmi des atomes d'halogènes et des groupes alcoxy, ou $R^2$, et $R^3$ ont chacun indépendamment une des significations ci-dessus et $R^4$ et $R^5$ représent ensemble un groupe alcoylène; et X représente un atome d'oxygène ou de soufre.

2. Un composé selon la revendication 1, dans lequel, R représente un atome d'hydrogène, un group alcoyle ayant jusqu'à 6 atomes de carbone qui peut être non-substitué ou peut être substitué par jusqu'à 3 atomes d'halogènes, un groupe cycloalcoyle ou un groupe benzyle qui peut être non-substitué ou peut être substitué dans le noyau par jusqu'à 3 atomes d'halogène.

3. Un composé selon l'une des revendications 1 et 2, dans lequel $R^1$ représente un atome d'hydrogène ou un groupe alcoyle ayant jusqu'à 6 atomes de carbone qui peut être non-substitué ou peut être substitué par jusqu'à 3 atomes d'halogènes.

4. Un composé selon l'une des revendications 1 à 3, dans lequel $R^2$, $R^3$, $R^4$ et $R^5$ représentent chacun indépendamment un atome dhydrogène, de fluor, de chlore ou de brome ou un groupe alcoyle ou alcényle ayant jusqu'à 4 atomes de carbone qui peut être non-substitué ou peut être substitué par jusqu'à 3 atomes de fluor, de chlore ou de brome, ou $R^2$ et $R^3$ ont chacun indépendamment une de ces significations et $R^4$ et $R^5$ représentent ensemble un groupe alcoylène ayant 4 ou 5 atomes de carbone.

5. Un composé selon l'une des revendications 1 à 4, dans lequel X représente un atome d'oxygène.

6. Un composé selon la revendication 1, qui est la 2-cyano-3-azabicyclo(3.1.0)hexan-4-one.

7. Un procédé pour la préparation d'un composé tel que revendiqué dans l'une des revendications 1 à 6, caractérisé en ce qu'un composé de la formule générale:

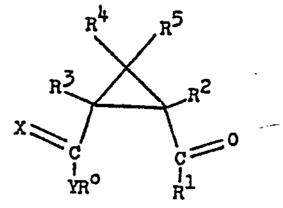

(II)

dans laquelle X, $R^1$, $R^2$, $R^3$, $R^4$, et $R^5$ ont les significations indiquées pour la formule générale I, Y représente un atome d'oxygène ou de soufre, R° représente un groupe alcoyle éventuellement substitué, un ion de métal alcalin, un ion d'ammonium éventuellement substitué par un ou plusieurs

groupes alcoyle, ou un équivalent d'un ion demétal alcalino-terreux, et les groupes —COR¹ et —CXYR° sont en relation cis l'un par rapport à l'autre, est mis à réagir avec un cyanure en présence d'un composé de la formule générale:

$$RNH_2 \quad (III)$$

dans laquelle R a la signification indiquée pour la formule générale I de la revendication 1.

8. Un procédé selon la revendication 7, caractérisé en ce que l cyanure est de l'acide cyanhydrique, un cyanure de métal alcalin, du cyanure d'ammonium, un cyanure d'ammonium dans lequel l'ion d'ammonium est substitué par un ou plusieurs groupes alcoyle, ou un cyanhydrine d'un aldéhyde ou d'une cétone.

9. Un procédé selon l'une des revendications 7 et 8, caractérisé en ce que, dans le composé de la formule générale II, R° représente un groupe alcoyle ayant jusqu'à 6 atomes de carbone, un ion de métal alcalin ou un ion d'ammonium éventuellement substitué par un ou plusieurs groupes alcoyle ayant de 1 à 4 atomes de carbone et Y représente un atome d'oxygène.

10. Un procédé selon l'une des revendications 7 à 9, caractérisé en ce que la réaction est conduite à une température comprise entre 0 et 100°C.

11. Un procédé selon l'une des revendications 7 à 10, caractérisé en ce que la réaction est conduite en la présence simultanée d'une base autre que le composé de la formule générale III.

**Patentansprüche**

1. Verbindung der allgemeinen Formel:

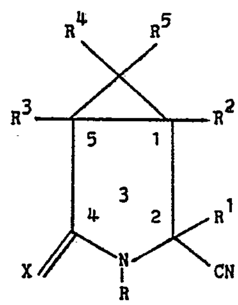

(I)

worin R ein Wasserstoffatom oder eine Alkyl- oder Cycloalkylgruppe, die gegebenenfalls durch ein bzw. eine oder mehrere gleiche oder verschiedene Halogenatome und/oder Alkoxy- oder Arylgruppen substituiert sein kann, vertritt, während $R^1$ ein Wasserstoffatom oder eine gegebenenfalls durch ein bzw. eine oder mehrere gleiche oder verschiedene Halogenatome und/oder Alkoxygruppen substituierte Alkyl- oder Cycloalkylgruppen darstellt, und worin $R^2$, $R^3$, $R^4$ und $R^5$ unabhängig von-einander je ein Wasserstoff- oder Halogenatom oder eine Alkyl-, Cycloalkyl-, Alkenyl-, Aryl, Alkaryl- oder Aralkylgruppe, die gegebenenfalls durch ein bzw. eine oder mehrere gleiche oder verschiedene Halogenatome und/oder Alkoxygruppen substituiert sein kann, vertreten oder worin $R^2$ und $R^3$ unabhängig von-einander eine der obigen Bedeutungen haben, während $R^4$ und $R^5$ gemeinsam eine Alkylengruppe vertreten; und worin X für ein Sauerstoff oder Schwefelatom steht.

2. Verbindung nach Anspruch 1, dadurch gekennzeichnet, dass R ein Wasserstoffatom, eine gegebenenfalls mit bis zu 3 Halogenatomen substituierte Alkylgruppe mit bis zu 6 Kohlenstoffatomen, eine Cycloalkylgruppe oder eine Benzylgruppe, die unsubstituiert oder im Ring mit bis zu 3 Halogenatomen substituiert sein können, vertritt.

3. Verbindung nach Anspruch 1 oder 2, dadurch gekennzeichnet, dass $R^1$ ein Wasserstoffatom oder eine gegebenenfalls mit bis zu 3 Halogenatomen substituierte Alkylgruppe mit bis zu 6 Kohlenstoffatomen vertritt.

4. Verbindung nach einem der Ansprüche 1 bis 3, dadurch gekennzeichnet, dass $R^2$, $R^3$, $R^4$ und $R^5$ unabhängig von-einander je ein Wasserstoff-, Fluor-, Chlor- oder Bromatom oder eine gegebenenfalls durch bis zu 3 Fluor-, Chlor- oder Bromatome substituierte Alkyl- oder Alkenylgruppe mit bus zu 4 Kohlenstoffatomen darstellen oder dass $R^2$ und $R^3$ jeweils unabhängig von-einander eine dieser Bedeutungen haben, während $R^4$ und $R^5$ gemeinsam eine Alkylengruppe mit 4 oder 5 Kohlenstoffatomen vertreten.

5. Verbindung nach einem der Ansprüche 1 bis 4, dadurch gekennzeichnet, dass X ein Sauerstoffatom bedeutet.

6. Verbindung nach Anspruch 1, welche Verbindung 2-Cyano-3-azabicyclo-[3,1,0]-hexan-4-on ist.

7. Verfahren zur Herstellung einer der in einem der Ansprüche 1 bis 6 beanspruchten Verbindungen, dadurch gekennzeichnet, dass man eine Verbindung der allgemeinen Formel:

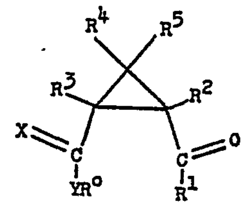

(II)

worin X, $R^1$, $R^2$, $R^3$, $R^4$ und $R^5$ die für die allgemeine Formel I angegebene Bedeutung haben, Y ein Schwefel- oder Sauerstoffatom ist und $R^o$ eine gegebenenfalls substituierte Alkylgruppe, ein Alkalimetallion, ein gegebenenfalls durch eine oder mehrere Alkylgruppen substituiertes Ammonium ion oder ein Äquivalent eines Erdalkaliions bedeutet, und worin die Gruppen —$COR^1$ und —$CXYR^o$ in cis-Konfiguration zu einander vorliegen, in Anwesenheit einer Verbindung der allgemeinen Formel:

$$RNH_2 \qquad (III)$$

worin R die in der allgemeinen Formel I des Anspruches 1 angegebene Bedeutung hat, mit einem Cyanid umsetzt.

8. Verfahren nach Anspruch 7, dadurch gekennzeichnet, dass man als Cyanid Cyanwasserstoff, ein Alkalicyanid, Ammoniumcyanid, ein Ammoniumcyanid, bei dem das Ammoniumion durch eine oder mehrere Alkylgruppen substituiert ist, oder ein Cyanhydrin eines Aldehydes oder Ketons verwendet.

9. Verfahren nach Anspruch 7 oder 8, dadurch gekennzeichnet, dass in der allgemeinen Formel II $R^o$ eine Alkylgruppe mit bis zu 6 Kohlenstoffatomen, ein Alkalimetallion oder ein gegebenenfalls durch eine oder mehrere Alkylgruppen mit 1 bis 4 Kohlenstoffatomen substituiertes Ammoniumion bedeutet, während Y ein Sauerstoffatom ist.

10. Verfahren nach einem der Ansprüche 7 bis 9, dadurch gekennzeichnet, dass man die Umsetzung bei einer Temperatur von 0 bis 100°C durchführt.

11. Verfahren nach einem der Ansprüche 7 bis 10, dadurch gekennzeichnet, dass man die Umsetzung in gleichzeitiger Anwesenheit einer Base durchführt, deren Konstitution nicht der Formel III entspricht.